# EUROPÄISCHE PATENTSCHRIFT

(11) **EP 2 532 317 B1**
(45) Veröffentlichungstag und Bekanntmachung des Hinweises auf die Patenterteilung: **25.11.2015**
(21) Anmeldenummer: 12157828.0
(22) Anmeldetag: 02.03.2012
(51) Int. Cl.: A61B 17/34, A61B 17/02

(54) **Aufhebelschutz für ein medizinisches Instrument zum Schaffen eines Zugangs für einen minimalinvasiven Eingriff**
Interlocking element for a medical instrument for creating an access point for minimally invasive procedures
Vérouillage pour un instrument médical destiné à créer un accès pour une intervention mini-invasive

(30) Priorität: 06.06.2011 DE 102011103526
(43) Veröffentlichungstag der Anmeldung: 12.12.2012
(73) Patentinhaber: Karl Storz GmbH & Co. KG, 78532 Tuttlingen (DE)
(72) Erfinder: Oberländer, Martin, 78234 Engen (DE); Sauer, Michael, 78532 Tuttlingen (DE); Beysang, Vincent, 68970 Guémar (FR)
(74) Vertreter: Witte, Weller & Partner Patentanwälte mbB

(56) Entgegenhaltungen:
- EP-A1- 2 228 024
- DE-A1- 2 801 696
- DE-A1-102009 014 524

## Beschreibung

Die Erfindung betrifft ein medizinisches Instrument zum Schaffen eines Zugangs für einen minimalinvasiven Eingriff, mit einem aus zumindest zwei Teilkörpern über deren Längskanten zusammengesetzten Hohlkörper, wobei jeder Teilkörper einen distalen Teilkörperabschnitt und einen davon abgewinkelten proximalen Teilkörperabschnitt aufweist, wobei ferner an den Längskanten Vorsprünge vorhanden sind, die mit Ausnehmungen an einer gegenüberliegenden Längskante in Eingriff stehen, und wobei die zusammengesetzten proximalen Teilkörperabschnitte durch eine Kappe zusammengehalten sind.

Ein derartiges medizinisches Instrument ist aus der EP 2 228 024 A1, sowie auch aus der DE102009014524 bekannt.

In einer ersten Position sind die zumindest zwei Teilkörper so zusammengesetzt, dass die jeweils distalen Teilkörperabschnitte einen etwa stabförmigen distalen Körper ergeben. In dieser Position kann das medizinische Instrument z.B. an einer Inzision in der Haut einer Bauchdecke angesetzt und durch die Bauchdecke hindurch bis in die Bauchhöhle eingetrieben werden. Somit funktioniert das medizinische Instrument in diesem Bauzustand wie eine Trokarhülse eines Trokars, der für einen laparoskopischen Eingriff eingesetzt wird.

Die proximalen Teilkörperabschnitte, die jeweils gegenüber den distalen Teilkörperabschnitten seitlich abgewinkelt sind, erstrecken sich seitlich von diesem distalen zusammengesetzten Körper weg und erheben sich über der Haut bzw. der Bauchdecke. Diese proximalen Teilkörperabschnitte werden anschließend so verschwenkt, dass diese sich zu einem proximalen Hohlkörper zusammenfügen. Dabei werden die im Bauchraum vorhandenen proximalen Teilkörperabschnitte seitlich voneinander weggeschwenkt. Auf den proximalen Hohlkörper wird dann eine dichtende Kappe aufgesetzt.

Bei dieser Schwenkbewegung rollen die gegenüberliegenden Längskanten der Teilkörper in dem gekrümmten Übergangsbereich zwischen dem jeweiligen distalen und dem jeweiligen proximalen Teilkörperabschnitt aneinander ab. Diese Abrollbewegung wird durch an den Längskanten vorhandene Vorsprünge, die in entsprechende Ausnehmungen an einer gegenüberliegenden Längskante in Eingriff treten können, geführt.

Diese Vorsprünge können Noppen oder Federn sein, die in entsprechende Vertiefungen bzw. Nuten in den gegenüberliegenden Längskanten eintreten.

Diese Noppen bzw. Nut-und-Federausgestaltung sorgt aber nicht nur für eine Führung dieses Abrollvorganges, sondern auch dazu, dass verhindert wird, dass die beiden Teilkörper, quer zu den Längskanten gesehen, sich voneinander trennen.

Zugleich wird durch das intensive Ineinandergreifen dieser Verzahnungsmerkmale ein relativ gasdichter Abschluss längs der Längskanten der aneinander gefügten proximalen Teilkörperabschnitte erzielt. Dadurch ist es möglich, wie das in der Laparoskopie üblich ist, in den Bauchraum durch den proximalen Hohlkörper, der durch die beiden proximalen Teilkörperabschnitte zusammengesetzt ist und durch die Kappe nach proximal abgeschlossen ist, ein Insufflationsgas einzuführen, um den Bauchraum aufzublähen.

Ein besonderer Vorteil des Instrumentes ist, dass ein durch dieses hindurchgeschobene Instrument in einem sehr großen Winkelbereich hin- und hergekippt werden kann. Dadurch eröffnet sich dem Operateur ein weites Operationsfeld für die durch das Instrument hindurchgeschobenen Operationsinstrumente, wie z.B. Endoskope, Scheren, Zangen und dergleichen, um einen operativen Eingriff durchzuführen.

Im praktischen Einsatz wurde nun festgestellt, dass bei extremen Kippstellungen von Instrumenten und einer entsprechenden Ausrichtung diese dazu neigen können, die beiden aneinandergefügten proximalen Teilkörperabschnitte etwas voneinander wegzubewegen, bzw. durch die Hebelwirkung des extrem gekippten Instrumentes diese auseinanderzuhebeln. Dabei wurde beobachtet, dass erhebliche Gasverluste durch Gasaustritte über die Längskanten der aneinandergefügten proximalen Teilkörperabschnitte auftreten, auch wenn diese nur Bruchteile von Millimetern auseinandergehebelt werden.

Aus dem deutschen Gebrauchsmuster G 78 01 125.3 ist ein Scheidenspekulum bekannt, das aus zwei halbschalenförmigen Teilkörpern zusammengesetzt ist, wobei jeder Teilkörper einen distalen Teilkörperabschnitt und einen davon abgewinkelten proximalen Teilkörperabschnitt aufweist.

Im Bereich der Krümmung, also des Überganges von dem jeweiligen distalen zu dem proximalen Teilkörperabschnitt, sind die beiden halbschalenförmigen Teilkörper über ein Gelenk fest miteinander verbunden. Bei aneinandergelegten distalen Teilkörperabschnitten kann das Spekulum in die Scheide eingeführt werden. Durch aneinanderfügen der proximalen Teilkörperabschnitte zu einem Hohlkörper spreizen dann die bereits eingeführten distalen Teilkörperabschnitte die Scheide auf. Der Arzt kann dann durch das als Hohlkörper ausgebildete Scheidenspekulum entsprechende visuelle Beobachtungen durchführen.

Damit die beiden proximalen Teilkörperabschnitte während des Beobachtens sich durch die Rückstellkraft des Scheidengewebes nicht wieder voneinander spreizen, ist eine Rastvorrichtung vorgesehen.

Die Rastvorrichtung weist an einem der beiden proximalen Teilkörper von dessen Längskanten in Umfangsrichtung vorstehende Laschen auf, die sich, im Querschnitt, vom freien Ende aus keilförmig verstärken. Beim gegenüberliegenden proximalen Teilkörperabschnitt sind an der Innenseite der Wand Ausnehmungen vorgesehen, in die die Laschen beim Zusammenführen eintreten und für einen sperrenden Eingriff sorgen.

Dazu muss aber die Geometrie und die Form der Laschen derart ausgebildet sein, dass diese eine solche Elastizität aufweisen, damit diese sich radial nach innen gerichtet verformen können, um in die Ausnehmungen an der Innenseite der Wandfläche des anderen proximalen Teilkörperabschnittes einfahren zu können.

Zum Trennen der Verrastung muss die Handhabungsperson den aus den beiden verrasteten proximalen Teilkörperabschnitten zusammengesetzten Hohlkörper durch starkes radiales nach innen Drücken etwas verformen, so dass die keilförmigen Laschen wieder aus den Ausnehmungen an der Innenwand austreten können. Bei einem Scheidenspekulum, das gegenüber dem Instrument der vorliegenden Anmeldung wesentlich größer ist und eine erhebliche Wandstärke aufweist und das meist aus metallischen Materialien hergestellt ist, können eine solche elastische Laschenkonstruktion einerseits und Ausnehmungen an der Innenwand des anderen proximalen Teilkörperabschnittes bewerkstelligt werden.

Bei wesentlich kleineren Hohlkörpern mit dünneren Wänden, wie das Instrument der vorliegenden Anmeldung, wird eine solche Konstruktion nicht betriebssicher arbeiten können. Es bestünde die Gefahr, dass durch extrem gekippte Instrumente Verformungen auftreten, die ein Austreten der keilförmigen Lasche aus der Ausnehmung an der Innenwand erlauben.

Es ist daher Aufgabe der vorliegenden Erfindung, ein Zugangsinstrument der eingangs genannten Art derart weiter zu entwickeln, dass ein Aufhebeln der aneinanderliegenden Längskanten der proximalen Teilkörper und ein damit verbundener Gasverlust effektiv vermieden wird.

Erfindungsgemäß wird die Aufgabe dadurch gelöst, dass zumindest ein Vorsprung in Bereich einer Längskante eines proximalen Teilkörperabschnittes als Haken ausgebildet ist, der sich von proximal nach distal derart erstreckt, dass er beim Abrollen der beiden Teilkörper während des Zusammenfügens der proximalen Teilkörperabschnitte sich von proximal nach distal bewegend in eine Ausnehmung an der gegenüberliegenden Längskante einfahrbar ist, und dadurch ein Aufhebeln der proximalen Teilkörperabschnitte sperrt, und dass der Haken bei einer entgegengesetzt gerichteten Abrollbewegung sich von distal nach proximal bewegend wieder aus der Ausnehmung ausfahrbar ist.

Man kann sich die Konstruktion als eine Art Reißverschluss vorstellen, bei der die riegelnden Elemente bei der Abrollbewegung, ohne dass Verformungen am Material zwingend notwendig sind, in die entsprechenden Ausnehmungen eintreten. Bei der entgegengesetzten Bewegung treten dann diese Riegelelemente wieder aus den Ausnehmungen aus. Also quasi wie wenn man einen Reißverschluss in einer Richtung schließt und in der entgegengesetzten Richtung öffnet.

Bei der Abrollbewegung bewegen sich die sich annähernden Längskanten der proximalen Teilkörperabschnitte aufeinander zu, wobei die Abrollachse, je nach Abrollzustand, im Bereich des gekrümmten Übergangsbereiches zwischen distalem Teilkörperabschnitt und proximalem Teilkörperabschnitt liegt.

Durch Vorsehen eines Hakens, der sich von proximal nach distal erstreckt, kann nun dieser Haken von "oben", also von proximal nach distal gesehen, in eine entsprechende Ausnehmung an der gegenüberliegenden Kante einfahren, ohne dass dabei Materialverformungen notwendig sind. Dasselbe erfolgt auch bei der entgegengesetzten Abrollrichtung, also wenn das Zugangsinstrument wieder vom Körper abgenommen werden soll, wozu die proximalen Teilkörperabschnitte wieder voneinander weggespreizt werden.

Der Eingriff eines solchen Hakens in eine Ausnehmung kann auch bei kleinen Bauteilen und auch bei Bauteilen aus Kunststoff eine ausreichende Widerstandskraft gegenüber Auseinanderhebeln der beiden proximalen Teilkörperabschnitte entgegen ihrer Zusammenfügrichtung sperren.

Somit kann durch konstruktiv einfache und sicher arbeitende konstruktive Merkmale auch bei kleinsten Bauteilen ein solches Aufhebeln und der damit verbundene Gasverlust sicher ausgeschlossen werden.

Es kann nur ein einziger solcher Haken oder es können auch über die Längskanten des proximalen Teilkörperabschnittes verteilt mehrere solche Haken vorhanden sein.

Ein Haken zeigt den Vorteil einer Zusammensetzhilfe beim Aneinanderfügen der Teilkörper. Der Haken unterscheidet sich von den anderen Vorsprüngen wie z.B. den Noppen und gibt dadurch eine eindeutige Orientierung und somit Hilfe, wie der eine Teilkörper an den anderen Teilkörper angesetzt und mit diesem zusammengefügt werden soll. Dadurch wird ein fehlerhaftes Ansetzen der Teilkörper im Sinne eines axialen Versatzes längs der Längskanten vermieden.

Das eigentliche Zusammenhalten der beiden Teilkörper wird durch die auf das proximale Ende der zusammengefügten proximalen Teilkörperabschnitte aufgesetzte Kappe bewerkstelligt.

Sinn und Zweck der vorliegenden Konstruktion ist es aber, im praktischen Einsatz, wenn also arbeitende Instrumente in dem medizinischen Instrument extrem gekippt werden, ein Aufhebeln der aneinanderliegenden proximalen Teilkörperabschnitte, insbesondere an von dem proximalen Ende weiter entfernt liegenden Bereichen zu verhindern und den damit verbundenen Gasaustritt zu vermeiden.

In einer weiteren Ausgestaltung der Erfindung ist der Haken als eine von der Längskante eines proximalen Teilkörperabschnittes vorspringende Nase ausgebildet, die in eine Ausnehmung in der gegenüberliegenden Längskante des anzufügenden proximalen Teilkörpers ein- und ausfahrbar ist.

Diese Maßnahme hat den Vorteil, dass die Nase als relativ stabiler abgerundeter Körper ausgebildet werden kann, der entsprechend sanft in die Ausnehmung bei den Abrollbewegungen einfährt oder austritt. Dabei kann man diese Nase auch bei relativ kleinen Bauteilen so stabil ausbilden, dass sie auch erheblichen Aufhebelkräften widerstehen kann.

In einer weiteren Ausgestaltung der Erfindung weist die Ausnehmung eine Hinterschneidung auf, hinter die der Haken einfahrbar ist.

Diese an sich bekannte Maßnahme hat den Vorteil, dass durch konstruktiv besonders einfache Mittel, nämlich durch eine Hinterschneidung, ein Abziehen des Hakens gesperrt werden kann.

In einer weiteren Ausgestaltung der Erfindung ist der Haken derart geformt, dass er in eingefahrenem Zustand bündig in der Ausnehmung liegt.

Diese Maßnahme hat nun mehrere Vorteile. Eine bündige Anlage zwischen Haken und Ausnehmung erlaubt eine relativ große Kontaktfläche, über die die Aufhebelkräfte verteilt werden können. Zugleich hat dies den Vorteil, dass im Bereich des Hakens selbst durch die bündige Anlage eine besonders gasdichte Verbindung zur Außenseite vorhanden ist.

In einer weiteren Ausgestaltung der Erfindung ist der Haken als ein Vorsprung der Längskante des einen proximalen Teilkörperabschnittes mit zumindest deren Wandstärke ausgebildet.

Diese Maßnahme hat den Vorteil, dass der Haken fertigungstechnisch besonders einfach herstellbar ist. Bei einem Spritzgussteil kann der Haken gleich als integraler Bestandteil der Wand des distalen Hohlkörpers hergestellt werden. Da dieser dann von der Längskante vorspringt kann er zumindest die Breite der Wandstärke aufweisen, gegebenenfalls kann er auch breiter sein, so dass ein besonders widerstandsfähiger und erhebliche Hebelkräfte aufnehmende Konstruktion gewährleistet ist.

In einer weiteren Ausgestaltung der Erfindung ist die Ausnehmung als eine Materialaussparung über die gesamte Wandstärke der Längskante eines proximalen Teilkörperabschnittes ausgebildet.

Auch diese Maßnahme hat wieder mehrere Vorteile. Fertigungstechnisch kann eine solche Ausnehmung sehr einfach hergestellt werden, bei einem Spritzgussteil bereits beim originären Spritzen des Teiles. Bei einem aus Metall hergestellten Körper kann diese Ausnehmung einfach ausgefräst werden. Auch hier ist es günstig, die Ausnehmung über die gesamte Materialbreite vorzusehen, so dass dann ein inniger Kontakt mit einem entsprechend ausgebildeten Haken möglich ist.

Wie bereits erwähnt, ist die Hauptaufgabe dieser Konstruktion, ein Lösen der beiden aneinandergefügten proximalen Hohlkörper entgegen der Zusammenfügrichtung zu sperren.

Quer zu den Längskanten sperren die weiteren, an der Längskante vorhandenen Bauteile, wie bspw. die Noppen oder die Nut- und Federkonstruktion.

In einer weiteren Ausgestaltung ist der Haken als einsetzbares Teil ausgebildet.

Diese Maßnahme hat den Vorteil, dass der Haken zunächst als separates Bauteil hergestellt werden kann und dann in den proximalen Teilkörperabschnitt eingesetzt werden kann. Dies eröffnet bspw. die Möglichkeit, diesen Haken aus anderen, insbesondere stabileren Materialien als das Material des proximalen Teilkörperabschnitts auszubilden. So kann bspw. der Haken als ein metallisches Teil ausgebildet werden, das dann in einen Körper aus Kunststoff eingesetzt werden kann, bspw. schon beim Spritzen des Kunststoffteiles mit integriert werden kann.

In einer weiteren Ausgestaltung der Erfindung ist die Ausnehmung in einem einsetzbaren Teil ausgebildet, das in die Wand eines proximalen Teilkörperabschnittes einsetzbar ist.

Hier ergibt sich prinzipiell derselbe Vorteil, das heißt, die Ausnehmung kann aus einem metallischen Einsatzstück ausgefräst werden und dieses dann in einen anderweitig vorgefertigten proximalen Teilkörperabschnitt eingesetzt werden. Dies kann bspw. auch wieder beim Kunststoffspritzen eines Kunststoffteils geschehen.

Besteht der Körper aus Metall, so ist die Ausnehmung einfach durch Ausfräsen von Material herstellbar.

Bei dem Körper aus metallischem Material müsste aus einem Vollmaterial so viel Material weggenommen werden, dass der Haken von der Wand vorstehend herausgearbeitet wird. Dies kann aufwändig und umständlich sein.

Daher könnte man bspw. den Haken als vorgefertigtes Stanzteil ausbilden und dann in eine entsprechende Ausnehmung in der Wand des proximalen Teilkörperabschnittes einsetzen.

Aufgrund des Reißverschlussprinzips kann der Haken vorteilhafterweise verformungsfrei in die Ausnehmung ein- und ausfahren.

Dadurch können Materialermüdungen oder Risse im Bereich des Hakens ausgeschlossen werden, die dessen Widerstandskraft gegen die Aushebelkräfte negativ beeinflussen könnten.

In einer weiteren Ausgestaltung der Erfindung ist zumindest ein Haken im Bereich einer Krümmung in einem Übergangsbereich zwischen den Längskanten eines proximalen Teilkörperabschnittes zum entsprechenden distalen Teilkörperabschnitt angeordnet.

Ein Haken setzt, wenn er im gekrümmten Übergangsbereich einer Längskante zwischen distalem und proximalem Teilkörperabschnitt angeordnet ist, eine ausreichende Widerstandskraft den Hebelkräften entgegen, die ein Aufhebeln verursachen könnten. Dies deswegen, da ein gekipptes Instrument genau in diesem gekrümmten Bereich ansetzt bzw. angreift um diese Hebelkräfte auszuüben, wie das in den Zeichnungen dargestellt ist. Das gekippte Instrument setzt einerseits am proximalen Rand an und andererseits in dem gekrümmten Übergansbereich an. Der Hebelwirkung wird also genau an der Stelle durch den Haken widerstanden, an der die Hebelkräfte ansetzen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind.

Die Erfindung wird nachfolgend im Zusammenhang mit den beiliegenden Zeichnungen anhand eines ausgewählten Ausführungsbeispieles näher beschrieben und erläutert.

Es zeigen:
- Fig. 1: zwei Teilkörper eines erfindungsgemäßen Instrumentes, die in einer ersten Position zum Einführen des Instrumentes über die distalen Teilkörperabschnitte zusammengesetzt sind,
- Fig. 2: eine Seitenansicht des medizinischen Instruments von Fig. 1, nachdem dieses in einen Körper gesetzt worden ist und die beiden proximalen Teilkörperabschnitte zusammengefügt worden sind und eine proximal abschließende Kappe aufgesetzt worden ist,
- Fig. 3: einen Längsschnitt durch die beiden aneinandergefügten Teilkörperabschnitte von Fig. 1, wobei ein Zwischenzustand dargestellt ist, der einen Übergang von der Verschwenkposition von Fig. 1 zu Fig. 2 entspricht,
- Fig. 4: eine stark vergrößerte Darstellung des in Fig. 3 mit einem Kreis umgrenzten Bereiches,
- Fig. 5: eine der Fig. 3 entsprechende Schnittdarstellung, bei der die proximalen Teilkörperabschnitte, wie das auch in Fig. 2 ersichtlich ist, aneinandergefügt sind,
- Fig. 6: einen stark vergrößerten Bereich des in Fig. 5 mit einem Kreis umgrenzten Bereiches,
- Fig. 7: eine der Darstellung von Fig. 5 entsprechenden Schnitt, wobei dargestellt ist, dass ein stabförmiges Instrument eingeführt ist, das extrem gekippt ist und dazu neigt, die beiden Teilkörper aufzuhebeln, und
- Fig. 8: eine stark vergrößerte Darstellung des in Fig. 7 mit einem Kreis umgrenzten Bereiches.

Ein in den Figuren dargestelltes Ausführungsbeispiel eines erfindungsgemäßen medizinischen Instruments ist in seiner Gesamtheit mit der Bezugsziffer 10 bezeichnet.

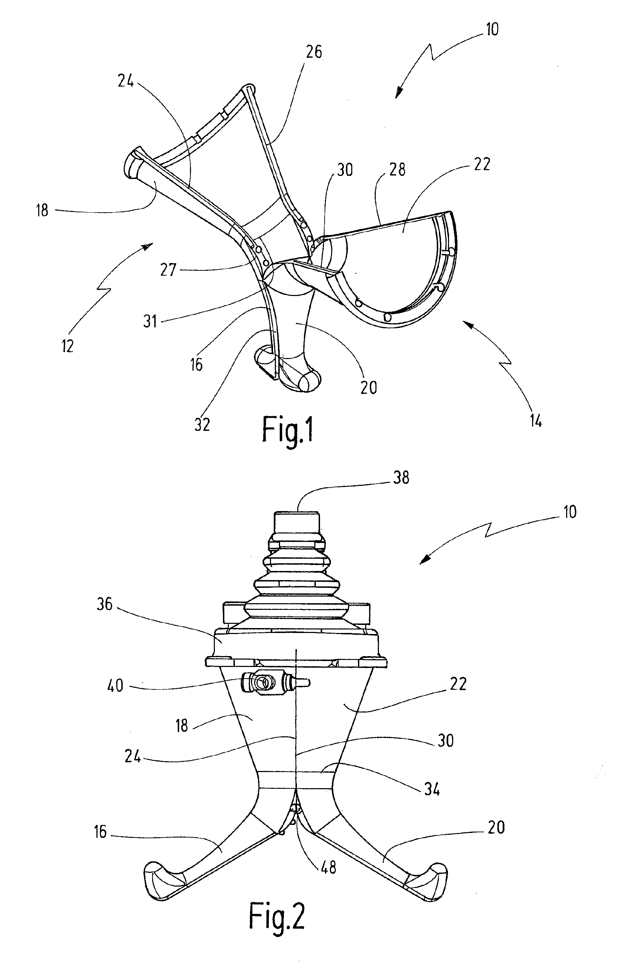

In Fig. 1 ist dargestellt, dass das medizinische Instrument 10 einen ersten Teilkörper 12 sowie einen zweiten Teilkörper 14 aufweist.

Der erste Teilkörper 12 weist einen distalen Teilkörperabschnitt 16 auf, von dem abgewinkelt ein proximaler Teilkörperabschnitt 18 absteht. Im Bereich des proximalen Teilkörperabschnitts 18 ist der erste Teilkörper 12 als halbschalenförmiger, sich nach proximal aufweitender, hohler Körper ausgebildet.

Auch der zweite Teilkörper 14 weist einen distalen Teilkörperabschnitt 22 und einen von diesem abgewinkelten proximalen Teilkörperabschnitt 24 auf. Auch dieser proximale Teilkörperabschnitt 24 ist wiederum als sich nach proximal aufweitender, halbschalenförmiger hohler Körper ausgebildet.

Die Größe und die Geometrie der beiden distalen Teilkörperabschnitte 16 und 22 ist derart, dass sie wenn sie wie in Fig. 1 dargestellt aneinandergefügt sind, einen distalen Körper 32 ergeben, der etwa stabförmig ausgebildet ist.

Aus Fig. 1 ist auch ersichtlich, dass der proximale Teilkörperabschnitt 18 zwei längs verlaufende Längskanten 24 und 26 aufweist. Diese Längskanten 24 und 26 verlaufen, von proximal gesehen, zunächst etwa geradlinig und gehen im Bereich einer Krümmung 27 in die entsprechenden geradlinigen Längskanten des distalen Teilkörperabschnittes 16 über.

Der proximale Teilkörperabschnitt 22 des zweiten Teilkörpers 14 weist ebenfalls zwei entsprechend ausgebildete Längskanten 28 und 30 auf, die über eine Krümmung 31 in die entsprechenden Längskanten des distalen Teilkörperabschnittes 20 übergehen. Die beiden aneinandergefügten Teilkörper 12 und 14 werden in dem in Fig. 1 dargestellten Bauzustand beim Setzen in dem Körper bspw. an einer Inzision einer Bauchdecke angesetzt und durch diese bis in den Bauchinnenraum eingetrieben, wie das an sich bekannt ist und insbesondere aus der eingangs erwähnten EP 2 228 024 A1 näher beschrieben ist.

Danach ragen noch die voneinander weggespreizten proximalen Teilkörperabschnitte 18 und 22 über die Hautfläche hinaus.

Die beiden aneinanderliegenden Teilkörper 12 und 14 werden nunmehr so umgeklappt, dass die beiden proximalen Teilkörperabschnitte 18 und 22 zu einem proximalen Hohlkörper 34 zusammengefügt werden, wie das in Fig. 2 dargestellt ist.

Bei dieser Bewegung rollen die beiden proximalen Teilkörperabschnitte 18 und 22 längs ihrer Längskanten 24 und 26 bzw. 28 und 30 aneinander ab.

Auf das obere proximale Ende der zusammengefügten proximalen Teilkörperabschnitte 18 und 22 wird dann eine abschließende Kappe 36 gesetzt, wie das aus Fig. 2 ersichtlich ist, die mehrere Funktionen aufweist.

Die Kappe 36 hält die beiden proximalen Teilkörperabschnitte 18 und 22 in dem in Fig. 2 dargestellten Bauzustand zu einem proximalen Hohlkörper 34 zusammen, da diese über den proximalen Rand der proximalen Teilkörperabschnitte 18 und 22 gestülpt ist. Zugleich sorgt sie für einen gasdichten Abschluss nach proximal. In der Kappe 36 ist zumindest ein Einlass 38 vorhanden, durch den ein Instrument durch die Kappe 36 und den distalen Hohlkörper 34 hindurch in das Innere des menschlichen oder tierischen Körpers eingeschoben werden kann, wie das an sich bekannt ist.

An der Außenseite des proximalen Teilkörperabschnittes 18 ist ein Gasanschluss 40 angebracht, über den ein Gas, meist CO₂, zum Aufblähen des Bauchinnenraums zugeführt werden kann.

Die Kappe 36 sorgt für einen dichten Abschluss nach proximal.

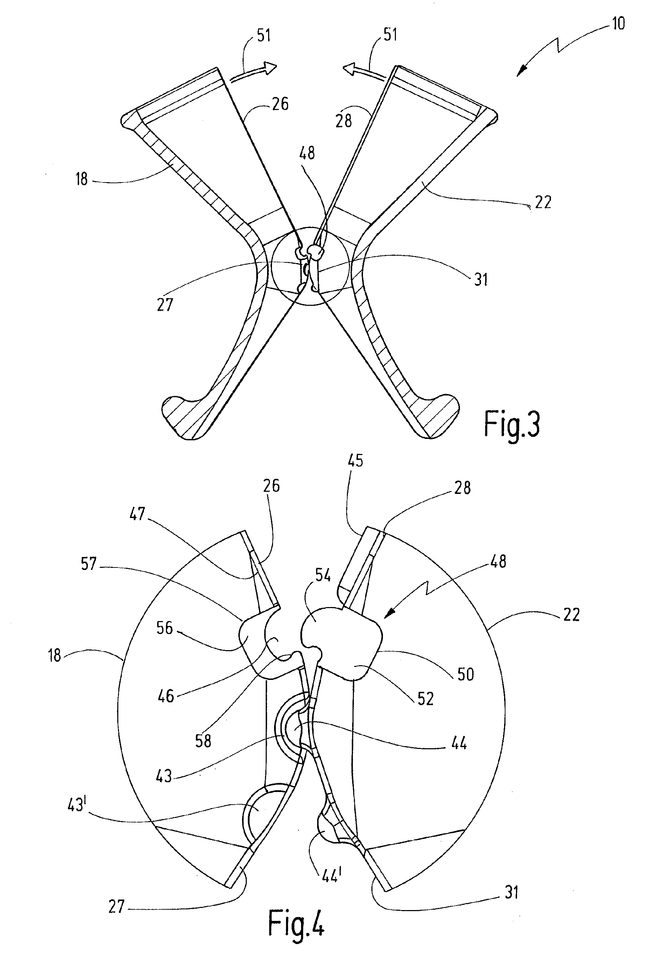

Problemstellen sind die aneinanderliegenden Längskanten 24 bzw. 30 der proximalen Teilkörperabschnitte sowie die entsprechend aneinanderliegenden Kanten 26 und 28. Um hier einen möglichst gasdichten Abschluss zu gewähren, ist, wie das insbesondere aus den Figuren 3 und 4 ersichtlich, an den Längskanten 26 und 28 eine Reihe von Verzahnungsmerkmalen vorgesehen. Insbesondere aus der vergrößerten Darstellung von Fig. 4 ist ersichtlich, dass von der Längskante 28 Noppen 44, 44' vorstehen, die in gegenüberliegende Vertiefungen 43, bzw. 43' der Längskante 26 eintreten können.

Im mehr proximalen Bereich steht von der Längskante 28 eine Art Feder 45 vor, die in eine hier nicht näher ersichtliche Längsnut 47 an der gegenüberliegenden Kante 26 eintreten kann.

Diese Konstruktion führt nicht nur zu einem gezielten und geführten Abrollen der Längskanten 28 und 26 beim Aneinanderfügen der proximalen Teilkörperabschnitte 18 und 20, sondern stellen auch zugleich Verzahnungsmerkmale dar, die ein Lösen der beiden proximalen Teilkörperabschnitte 18 und 22 quer zu der Längserstreckung der Längskanten sperren.

Insbesondere aus der vergrößerten Darstellung von Fig. 4 ist ersichtlich, dass im Bereich der Krümmung 31 der Längskante 28 des proximalen Teilkörperabschnittes 22 von dieser ein Haken 48 vorsteht, der in eine entsprechende Ausnehmung 46 an der gegenüberliegenden Längskante 26 des proximalen Teilkörperabschnittes 18 eintreten kann, wenn die Längskanten 26 und 28, wie das in Fig. 3 durch die beiden Pfeile 51 angedeutet ist, beim Zusammenfügen aufeinander zubewegt werden. Der Haken 48 weist dabei eine Nase 54 auf, die sich von proximal nach distal geneigt erstreckt.

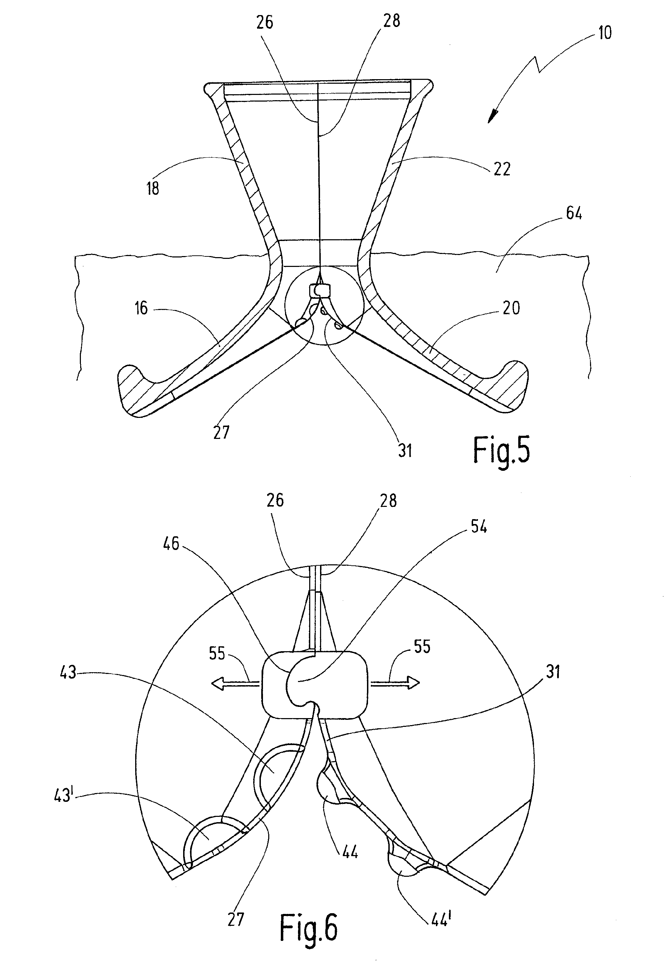

Die Ausnehmung 46 weist eine entsprechende Hinterschneidung 58 auf. Beim Abrollen und Zusammenfügen läuft nunmehr die Nase 54 des Hakens 48 in die Ausnehmung 46 und hinter deren Hinterschneidung 58 ein, wie das aus dem Übergang von Fig. 4 zu Fig. 6 ersichtlich ist.

Dabei ist keine Materialverformung notwendig, sondern die Nase 54 bzw. der Haken 48 läuft bei der Abrollbewegung längs der Krümmung 31 bzw. der Krümmung 27 passend in die Ausnehmung 46 und hinter die Hinterschneidung 58 hinein.

In diesem Zustand, der also dem in Fig. 2 bzw. in Fig. 5 und 6 dargestellten Zustand entspricht, sperrt somit der Haken 48 eine Bewegung der beiden aneinanderliegenden proximalen Teilkörperabschnitte 18 und 22 entgegen der Zusammenfügrichtung, wie das durch Pfeile 55 in Fig. 6 dargestellt ist.

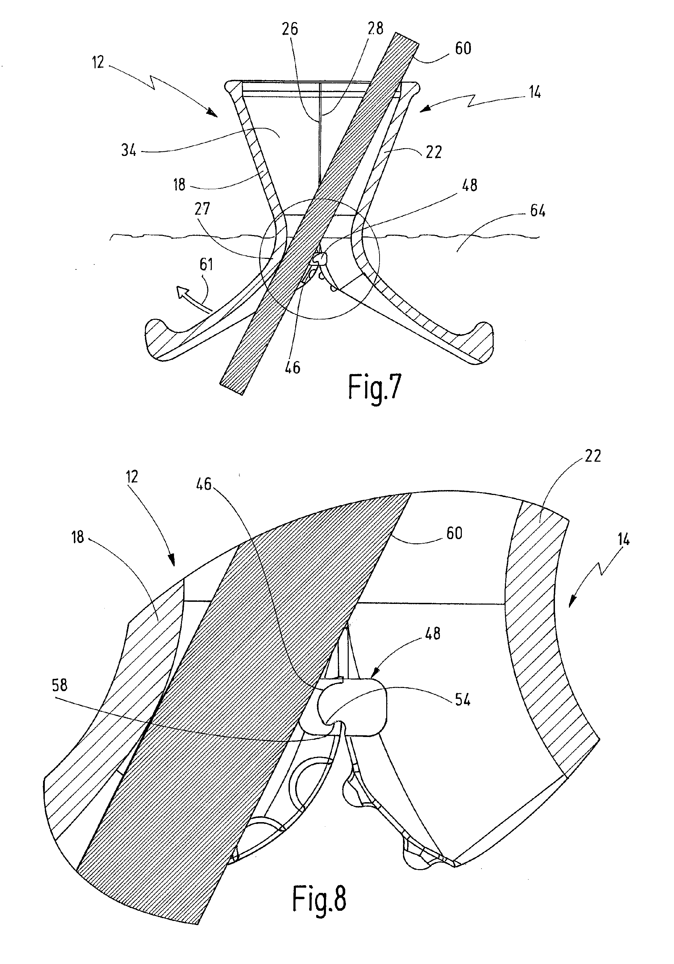

In den Figuren 7 und 8 ist eine Situation dargestellt, bei der solche Hebelkräfte auftreten können, die dazu neigen, die aneinandergefügten Längskanten 26 und 28 bei aufgesetzter Kappe 36 etwas voneinander wegzubewegen.

In Fig. 7 ist dargestellt, dass ein stabförmiges Instrument 60 ein- und durchgeführt ist und dass dieses extrem gekippt ist.

Dabei stößt es am proximalen Ende an den oberen proximalen Rand, hier bspw. des proximalen Teilkörperabschnittes 22.

Innen trifft dieses stark gekippte Instrument 60 auf den distalseitigen Endabschnitt des proximalen Teilkörperabschnittes 18 des anderen Teilkörpers 12 und neigt dazu, dieses, insbesondere im Bereich der Krümmung 27, auszuhebeln, also von der Längskante 28 wegzubewegen.

Wird eine solche Hebelkraft nicht aufgewendet, reicht die Rückstellkraft der Bauchdecke 64, durch die das erfindungsgemäße Instrument 10 hindurchgeschoben ist, normalerweise aus, um eine ausreichende Anpresskraft der beiden Teilkörper 12 und 14 in diesem Bereich zu sorgen, um Gasverluste zu vermeiden.

Wird aber, wie das in Fig. 7 dargestellt ist, ein Instrument so stark gekippt, besteht die Gefahr, dass die beiden Teilkörper 12 und 14 auseinandergehebelt werden.

Aus der vergrößerten Darstellung von Fig. 8 ist zu entnehmen, dass die Nase 54 des Hakens 48, der hinter die Hinterschneidung 58 eingefahren ist, genau diese Aushebelbewegung sperrt, insbesondere wenn er an dieser Stelle des gekrümmten Übergangsbereiches angeordnet ist.

Diese Konstruktion sperrt selbstverständlich auch, wenn das Instrument 60 in die entgegengesetzte Richtung verkippt wird.

In dem Ausführungsbeispiel ist nur ein einziger solcher Haken dargestellt, es können aber auch mehrere solche Haken im Bereich der Krümmung in Richtung proximalem Ende vorgesehen sein, falls zu befürchten ist, dass aufgrund der eingesetzten Instrumente extreme große Hebelmomente ausgeübt werden können.

In Fig. 4 ist angedeutet, dass der Haken 48 als ein separates Teil 50 ausgebildet sein kann, das in die Wand des proximalen Teilkörperabschnitts 22 im Bereich der Längskante 28 eingesetzt ist. Dazu weist dieser ein eckiges Einsatzstück 52 auf, das in eine entsprechende Ausnehmung oder Ausfräsung festsitzend eingesetzt ist. Dies kann durch Verschrauben, Verlöten oder auch durch Einspritzen bei einem Kunststoffspritzvorgang erfolgen.

Entsprechend ist an der gegenüberliegenden Längskante 26 ein Teil 56 eingesetzt, das die Ausnehmung 46 aufweist. Dies ist dann entsprechend eingesetzt oder verankert.

Dies eröffnet bspw. die Möglichkeit, die Teilkörper 12 und 14 aus einem kostengünstigen Kunststoffmaterial durch einen einfachen Spritzvorgang herzustellen. Für eine effektive Aufhebelsicherung und zur Aufnahme von erheblichen Kräften können dann die Teile 50 und 56 aus widerstandsfähigerem Material, z.B. aus Metall, hergestellt werden und als Einsatzstücke eingesetzt werden. Das kann bspw. schon beim originären Spritzvorgang erfolgen.

Dadurch kann schon ein einziger solcher Haken ausreichen, um eine ausreichende Hebelsicherung zu bewerkstelligen.

Es ist selbstverständlich auch möglich, diese Haken und Ausnehmung bei aus Kunststoff hergestellten Teilen direkt beim Spritzvorgang herzustellen.

Bei Instrumenten aus metallischem Material ist die Ausnehmung durch einen einfachen Fräsvorgang zu bewerkstelligen, der Haken müsste aber dann aus dem Vollmaterial herausgearbeitet oder entsprechend angesetzt werden. Hier stehen dem Fachmann zahlreiche Möglichkeiten zur Verfügung, je nach Kundenwunsch entsprechende Konstruktionen zur Verfügung zu stellen.

Aus Fig. 8 ist insbesondere zu erkennen, dass der Haken 48 bzw. dessen Nase 54 exakt der Kontur der Ausnehmung 46 entspricht, somit in dieser bündig aufgenommen ist.

Dies trägt zusätzlich zu einem gasdichten Abschluss in diesem Bereich bei, nämlich gegenüber einem Gasdurchtritt quer zur Längsrichtung der Längskanten 26 und 28 bzw. 24 und 30 im Bereich des Hakens 48.

## Patentansprüche

1. Medizinisches Instrument zum Schaffen eines Zugangs für einen minimalinvasiven Eingriff, mit einem aus zumindest zwei Teilkörpern (12, 14) über deren Längskanten (26, 28; 30, 32) zusammengesetzten Hohlkörper (34), wobei jeder Teilkörper (12, 14) einen distalen Teilkörperabschnitt (16, 20) und einen davon abgewinkelten proximalen Teilkörperabschnitt (18, 22) aufweist, wobei ferner an den Längskanten (26, 28; 30, 32) Vorsprünge (42) vorhanden sind, die mit Ausnehmungen (43) an einer gegenüberliegenden Längskante in Eingriff stehen, und wobei die zusammengesetzten proximalen Teilkörperabschnitte (18, 22) durch eine Kappe (36) zusammengehalten sind, **dadurch gekennzeichnet, dass** zumindest ein Vorsprung (42) im Bereich einer Längskante (28) eines proximalen Teilkörperabschnittes (27) als Haken (48) ausgebildet ist, der sich von proximal nach distal derart erstreckt, dass er beim Abrollen der beiden Teilkörper (12, 14) während des Zusammenfügens der proximalen Teilkörperabschnitte (18, 22) sich von proximal nach distal bewegend in eine Ausnehmung (46) an der gegenüberliegenden Längskante (26) einfahrbar ist und dadurch ein Aufhebeln der proximalen Teilkörperabschnitte (18, 22) sperrt, und dass der Haken (48) bei einer entgegengesetzt gerichteten Abrollbewegung sich von distal nach proximal bewegend wieder aus der Ausnehmung (46) ausfahrbar ist.

2. Medizinisches Instrument nach Anspruch 1, **dadurch gekennzeichnet, dass** der Haken (48) eine von der Längskante (28) eines proximalen Teilkörperabschnittes (22) vorspringende Nase (54) aufweist, die in eine Ausnehmung (46) in der gegenüberliegenden Längskante (26) des anzufügenden proximalen Teilkörpers ein- und ausfahrbar ist.

3. Medizinisches Instrument nach Anspruch 1 oder 2, **dadurch gekennzeichnet, dass** die Ausnehmung (46) eine Hinterschneidung (58) aufweist, hinter die der Haken (48) einfahrbar ist.

4. Medizinisches Instrument nach einem der Ansprüche 1 bis 3, **dadurch gekennzeichnet, dass** der Haken (48) derart geformt ist, dass er in eingefahrenem Zustand bündig in der Ausnehmung (46) liegt.

5. Medizinisches Instrument nach einem der Ansprüche 1 bis 4, **dadurch gekennzeichnet, dass** der Haken (48) als ein Vorsprung der Längskante (28) des einen proximalen Teilkörperabschnittes (22) mit zumindest deren Wandstärke ausgebildet ist.

6. Medizinisches Instrument nach einem der Ansprüche 1 bis 5, **dadurch gekennzeichnet, dass** die Ausnehmung (46) eine Materialaussparung über die gesamte Wandstärke der Längskante eines proximalen Teilkörperabschnittes (18) ausgebildet ist.

7. Medizinisches Instrument nach einem der Ansprüche 1 bis 6, **dadurch gekennzeichnet, dass** der Haken (48) als einsetzbares Teil (50) ausgebildet ist.

8. Medizinisches Instrument nach einem der Ansprüche 1 bis 7, **dadurch gekennzeichnet, dass** die Ausnehmung (46) in einem einsetzbaren Teil (56) ausgebildet ist, das in die Wand eines proximalen Teilkörperabschnittes (18) einsetzbar ist.

9. Medizinisches Instrument nach einem der Ansprüche 1 bis 8, **dadurch gekennzeichnet, dass** der Haken (48) verformungsfrei in die Ausnehmung (46) ein und ausfahrbar ist.

10. Medizinisches Instrument nach einem der Ansprüche 1 bis 9, **dadurch gekennzeichnet, dass** zumindest ein Haken (48) im Bereich einer Krümmung (27, 31) in einem Übergangsbereich zwischen den Längskanten (24, 26; 28, 30) eines proximalen Teilkörperabschnittes (18; 22) zum entsprechenden distalen Teilkörperabschnitt (16; 20) angeordnet ist.

## Claims

1. Medical instrument for creating an access for a minimally invasive intervention, with a hollow body (34) assembled from at least two subsidiary bodies (12, 14) along the longitudinal edges (26, 28; 30, 32) thereof, wherein each subsidiary body (12, 14) has a distal subsidiary body portion (16, 20) and, at an angle to the latter, a proximal subsidiary body portion (18, 22), and wherein projections (42) are present on the longitudinal edges (26, 28; 30, 32) and engage with recesses (43) on a opposite longitudinal edge, and wherein the assembled proximal subsidiary body portions (18, 92) are hold together via a cap (36), **characterized in that** at least one projection (42) in the area of a longitudinal edge (28) of a proximal subsidiary body portion (27) is designed as a hook (48), which extends from proximal to distal in such a way that, in a rolling movement of the two subsidiary bodies (12, 14) during the joining-together of the proximal subsidiary body portions (18, 22) enters from proximal to distal into a recess (46) on the opposite longitudinal edge (26) and thus block a pulling-apart of the proximal subsidiary body portions (18, 22), and **in that** the hook (48) moves out of the recess (46) from distal to proximal during an oppositely directed rolling movement.

2. Medical instrument of claim 1, **characterized in that** the hook (48) has a lug (54) which projects from the longitudinal edge (28) of a proximal subsidiary body portion (22) and which can be driven in and out of a recess (46) in the opposite longitudinal edge (26) of the proximal subsidiary body that is to be joined.

3. Medical instrument of claims 1 or 2, **characterized in that** the recess (46) has an undercut (58), behind which the hook (48) can be inserted.

4. Medical instrument of anyone of claims 1 through 3, **characterized in that** the hook (48) is shaped in such a way, that, when it has been inserted, it lies flush in the recess (46).

5. Medical instrument of anyone of claims 1 through 4, **characterized in that** the hook (48) is designed as a projection on the longitudinal edge (28) of one proximal subsidiary body portion (22), with at least the wall thickness of the latter.

6. Medical instrument of anyone of claims 1 through 5, **characterized in that** the recess (46) is designed as a material cutout through the entire wall thickness of the longitudinal edge of a proximal subsidiary body portion (18).

7. Medical instrument of anyone of claims 1 through 6, **characterized in that** the hook (48) is designed as an insertable part (50).

8. Medical instrument of anyone of claims 1 through 7, **characterized in that** the recess (46) is formed as an insertable part (56) that can be inserted into the wall of a proximal subsidiary body portion (18).

9. Medical instrument of anyone of claims 1 through 8, **characterized in that** the hook (48) can be moved without deformation in and out of the recess (46).

10. Medical instrument of anyone of claims 1 through 9, **characterized in that** at least one hook (48) is arranged in the area of a curvature (27, 31) in a transition area between the longitudinal edges (24, 26; 28, 30) of a proximal subsidiary body portion (18; 22) and the corresponding distal subsidiary body portion (16; 20).

## Revendications

1. Instrument médical destiné à créer un accès pour une intervention mini-invasive, doté d'un corps creux (34) composé d'au moins deux corps de parties (12, 14) s'assemblant au niveau de leurs arêtes longitudinales (26, 28 ; 30, 32), où chaque corps de partie (12, 14) présente une section de corps de partie distale (16, 20) et une section de corps de partie proximale (18, 22) qui est coudée par rapport à cette dernière, où en outre des parties en saillie (42) sont en outre présentes au niveau des arêtes longitudinales (26, 28 ; 30, 32), lesquelles sont en prise avec des creux (43) au niveau d'une arête longitudinale située en face, et où les sections de corps de parties proximales assemblées (18, 22) sont maintenues ensemble par une coiffe (36), **caractérisé en ce qu'**au moins une partie en saillie (42) dans la zone d'une arête longitudinale (28) d'une section de corps de partie proximale (27) est conçue sous forme de crochet (48), lequel s'étend de l'extrémité proximale vers l'extrémité distale de telle sorte qu'il peut être introduit dans un creux (46) au niveau de l'arête longitudinale (26) située en face par rotation des deux corps de parties (12, 14) pendant l'assemblage des sections de corps de parties proximales (18, 22) en allant de l'extrémité proximale vers l'extrémité distale et grâce à quoi une levée des sections de corps de parties proximales (18, 22) est bloquée, et **en ce que** le crochet (48) peut être extrait hors du creux (46) par un mouvement de rotation dans le sens opposé en revenant de l'extrémité distale vers l'extrémité proximale.

2. Instrument médical selon la revendication 1, **caractérisé en ce que** le crochet (48) présente un nez en saillie (54) depuis l'arête longitudinale (28) d'une section de corps de partie proximale (22), lequel peut être introduit dans un creux (46) dans l'arête longitudinale située en face (26) du corps de partie proximal à assembler et en être extrait.

3. Instrument médical selon la revendication 1 ou 2, **caractérisé en ce que** le creux (46) présente une contre-dépouille (58), derrière laquelle le crochet (48) peut être introduit.

4. Instrument médical selon l'une des revendications 1 à 3, **caractérisé en ce que** le crochet (48) est façonné de telle sorte qu'il se trouve dans un état rétracté à fleur dans le creux (46).

5. Instrument médical selon l'une des revendications 1 à 4, **caractérisé en ce que** le crochet (48) est conçu sous la forme d'une partie en saillie de l'arête longitudinale (28) d'une section de corps de partie proximale (22) avec au moins son épaisseur de paroi.

6. Instrument médical selon l'une des revendications 1 à 5, **caractérisé en ce que** le creux (46) est conçu par un dégagement de matière sur l'ensemble de l'épaisseur de paroi globale d'une arête longitudinale d'une section de corps de partie proximale (18).

7. Instrument médical selon l'une des revendications 1 à 6, **caractérisé en ce que** le crochet (48) est conçu sous la forme d'une partie insérable (50).

8. Instrument médical selon l'une des revendications 1 à 7, **caractérisé en ce que** le creux (46) est ménagé dans une partie insérable (56), laquelle est insérable dans la paroi d'une section de corps de partie proximale (18).

9. Instrument médical selon l'une des revendications 1 à 8, **caractérisé en ce que** le crochet (48) peut être introduit dans le creux (46) et en être extrait sans déformation.

10. Instrument médical selon l'une des revendications 1 à 9, **caractérisé en ce qu'**au moins un crochet (48) est agencé dans la zone d'une courbure (27, 31) dans une zone de transition entre les arêtes longitudinales (24, 26 ; 28, 30) d'une section de corps de partie proximale (18; 22) vers les sections de corps de parties distales correspondantes (16 ; 20).
